# EUROPEAN PATENT SPECIFICATION

(11) **EP 3 042 607 B1**
(45) Date of publication and mention of the grant of the patent: **04.09.2024**
(21) Application number: 15150843.9
(22) Date of filing: 12.01.2015
(51) Int. Cl.: A61B 5/30

(54) **Biopotential signal acquisition system and method**
Biopotenzialsignalerfassungssystem und -verfahren
Système d'acquisition de signal biopotentiel et procédé

(43) Date of publication of application: 13.07.2016
(73) Proprietor: Stichting IMEC Nederland, 5656 AE Eindhoven (NL); IMEC VZW, 3001 Leuven (BE)
(72) Inventor: Xu, Jiawei, 3001 Leuven (BE); Yazicioglu, Refet Firat, 3001 Leuven (BE)
(74) Representative: Patent Department IMEC

(56) References cited:
- WO-A1-2014/043739
- US-A- 3 885 552
- US-A- 5 813 993
- US-A1- 2012 095 361

## Description

### Technical Field

This invention relates to the acquisition of biopotential signals. The invention is for example of interest for ECG (electrocardiography), EEG (electroencephalography), EMG (electromyography) or EOG (electrooculogram) signals.

### Background

Biopotentials are usually recorded using electrodes attached to the body, such as wet (gel) electrodes, or dry electrodes. The electrodes are used to measure biopotentials, which typically have a magnitude in the range 1µV to 10mV.

Active electrodes have been employed in biopotential acquisition systems, in which the electrodes are integrated with amplifiers for the suppression of interference picked up from cables. The active electrode based system is robust to cable motion artifacts and interferences, which makes it suitable for dry electrode applications.

An ideal active electrode comprises a passive electrode and a pre-amplifier that are integrated within the same package or board, which can be placed very close to the skin to extract the low-level biopotential signals. In this way, the signal path length between the electrode and the pre-amplifier is minimized, maintaining the highest possible input impedance of the amplifier and lowest possible noise pick-up from electromagnetic fields. Furthermore, the output of the active electrode forms a low-impedance node and the interference and motion artifacts obtained by cable movement and electromagnetic fields in the vicinity can both be reduced when compared to a conventional passive electrode interface.

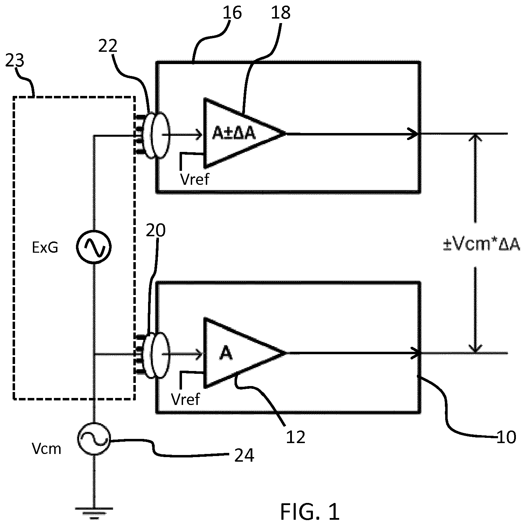

**Figure 1** shows the basic active electrode system. A reference active electrode 10 comprises an amplifier 12. Contact is made with the patient at a first contact 20 which provides an input to the system. A first signal active electrode 16 comprises an amplifier 18. Contact is made with the patient at a second contact 22. The gain of the reference active electrode amplifier is shown as A and the gain of the signal active electrode amplifier is shown as A+ΔA. Thus, there is a gain mismatch between the active electrodes. Note that for a digitally interfaced system, analogue to digital converter units may be implemented as part of the electrodes 10,16 or they may be implemented downstream from the electrodes.

Common mode interference is one of the major problems in such active electrode biopotential signal acquisition systems. For example, biopotential signals can be affected by interference currents derived from the mains power supply lines, known as "common mode aggressors". The mains frequency generally falls within the frequency range of interest of biomedical signals, which makes such common mode signals a particular problem. For example, an ECG signal has its main frequency components in the range 0.5Hz to 40Hz, but signal information up to around 100Hz or even 200Hz is desired.

The signals generated by the patient side are shown in region 23. The common mode interference is represented by the signal source 24 in Figure 1. The patient generates the signal ExG to be monitored between the reference electrode and the signal electrode. The notation ExG is used to indicate any of EEG, ECG, EMG or EOG signals.

The interference signals can have larger amplitudes than the biopotential signals to be measured. This requires biopotential amplifier to have very high common mode rejection ratio (CMRR) in order to prevent the common mode interference signals from converting into differential signals, appearing at the output of the amplifier, and thereby reject the 50 Hz or 60 Hz common mode interferences whilst extracting the biopotential signals. When a high common mode rejection ratio is not possible, notch filtering is normally required to remove the common mode interfering signal.

A particular problem is that the common mode interference at the inputs of the active electrodes can be converted to a differential mode error at the outputs of active electrode pairs due to the voltage gain mismatch between the active electrode pairs, as well as the contact-impedance mismatch. The output error can have significant amplitude when compared to the amplitude of the biopotential signals. The CMRR of active electrode systems is usually limited by this voltage gain mismatch (VCM *ΔA) between the signal active electrode and the reference active electrode, which in return is due to the process variation and component mismatch. The process variation and component mismatch make it difficult to achieve well-matched voltage gain. Without compensation circuits or trimming, achieving more than 60dB CMRR is very difficult because of the component mismatch, while medical grade biopotential systems typically require more than 80dB CMRR.

A large common mode input interference may also saturate the active electrode, especially when the amplifier has a large voltage gain, and the other input is connected to a reference voltage. To avoid saturation of the active electrode, its voltage gain is usually limited to accommodate large input common mode dynamic range.

The article "A wearable 8-channel active-electrode EEG/ETI acquisition system for body area networks", by Jiawei Xu et. al., in IEEE Journal of Solid State Circuits, col. 49, no. 9, September 2014 discloses the use of a feedforward approach for cancellation of a common mode interferer. The common mode feedforward scheme is implemented inside each of the active electrodes. There are limitations to the use of the circuit, in particular it is only suitable for certain amplifier designs.

Another known system for the measurement of physiological signals using active electrodes is described in WO 2014/043739 A1, which includes a sensing element for sensing a biological signal generated in a subject's body, the biological signal including a noise component and an information carrying component: a filter module separates the noise component and the information carrying component of the biological signal from each other, the filter module generating a filtered signal from the biological signal input into the filter module; an amplifier has an inverting input and a non-inverting input, the filter module being connected to one of the inputs of the amplifier for receiving the filtered signal and the sensed biological signal being input to the other of the inputs so at least the noise component of the biological signal is attenuated relative to the information carrying component.

### Summary

The invention is defined by the claims.

According to the invention, there is provided a biopotential signal acquisition system as in claim 1.

According to an exemplary embodiment, the integrated amplifier of each active electrode (which functions as a pre-amplifier, as there will generally be further amplification in the signal processing path) is connected to a feedforward common mode voltage provided by the unity gain voltage buffer. This buffered signal is approximately equal to the input common mode signal, which can be used to bias the other input of the amplifier. As a result, the effective common-mode input voltage which appears at the input of each amplifier is significantly reduced. This reduces the common-mode gain of the two active electrodes, and thus improves their common mode rejection ratio. It also avoids the saturation of the active electrodes, but without affecting the differential amplification of the signal of interest. The system may be applied to a single channel system (with two active electrodes) or to a multiple channel system (with more than two active electrodes). The system can be used with different types of amplifier, including inverting and non-inverting amplifiers.

The first and second inputs are skin contact points at which electrode pads are applied to the skin of the patient being monitored. They may be wet (gel) or dry contacts, although as described above systems using dry electrode contacts are preferred by patients for reasons of ease of use.

The unity gain buffer may be for receiving a signal from an associated input which is additional to the first and second inputs. Alternatively, the unity gain buffer may be for receiving a signal from the first or second input. In this case, no additional contact electrodes are needed to those already required for the active electrodes. The unity gain buffer may for example be implemented as an operational amplifier with full negative feedback, i.e. the output connected directly to the inverting input, and the input signal from the skin contact provided to the non-inverting input.

There may be at least a third active electrode for receiving a signal from a third input and comprising a third integrated amplifier. Three active electrodes enable a two channel system. The unity gain buffer may then also provide the buffered output to the third integrated amplifier as a reference signal. Thus, the unity gain buffer may be shared between two or more active electrodes. There may be one unity gain buffer even for many active electrodes, but equally, there may be more than one unity gain buffer, each providing a reference signal to an associated set of active electrodes.

In one set of examples, the output of the unity gain buffer may be provided to the respective integrated amplifiers through decoupling capacitors. These prevent a dc signal from the unity gain buffer from saturating the active electrode amplifiers. The unity gain buffer output may be provided to inverting inputs of the respective integrated amplifiers through the decoupling capacitors, and the respective integrated amplifiers may then also comprise a negative feedback path. This feedback path may be used to stabilize the DC operating points of the active electrode amplifiers. This provides an implementation using inverting amplification of the input signals and decoupling of the buffer signal.

In another set of examples, the output of the unity gain buffer is provided to non-inverting inputs of the respective integrated amplifiers. The first and second inputs may then be provided to the respective integrated amplifiers through decoupling capacitors, and the respective integrated amplifiers may again comprise a negative feedback path. This provides an implementation using non-inverting amplification of the input signals, and decoupling of the input signals.

The first active electrode may comprise a reference electrode, and the second active electrode may comprise one of a set of active electrode. As a minimum, the set may comprise only one active electrode (for a one channel implementation). However, the set may for example comprise between 8 and 32 active electrodes (for 8 to 32 channels respectively).

The invention also provides a biopotential signal acquisition method as in claim 10.

This method provides a common mode feedforward approach in which a common mode signal may be fed forward from a unity gain buffer, which may be external to the active electrodes, to any number of active electrodes, in order to improve the common mode rejection ratio. The first, second and common mode biopotential signals may each be received from a respective input or else the first and second biopotential signals may each be received from a respective input and the common mode biopotential signal comprises one of the first and second biopotential signals.

Third or further biopotential signals may also be captured using a third or further active electrode comprising an associated integrated amplifier. The buffered output may also be provided to the third or further integrated amplifier as a reference common mode signal. In this way, the concept can be applied to single channel or multiple channel systems.

The buffered common mode biopotential signal or the first and second biopotential signals may be provided to the respective integrated amplifiers through decoupling capacitors to suppress DC signals between the inputs. A negative feedback path may be used to stabilize the operating points of the amplifiers.

### Brief description of the drawings

Examples of the invention will now be described in detail, with reference to the accompanying drawings, in which:
**Figure 1** shows schematically a known active electrode system for measuring biopotentials;
**Figure 2** shows a first example of signal acquisition system according to the invention;
**Figure 3** shows a second example of signal acquisition system according to the invention;
**Figure 4** shows a third example of signal acquisition system according to the invention;
**Figure 5** shows how patient signals can lead to amplifier saturation;
**Figure 6** shows a fourth example of signal acquisition system according to the invention addressing the problem explained with reference to Figure 5; and
**Figure 7** shows a fifth example of signal acquisition system according to the invention adressing the problem explained with reference to Figure 5.

### Detailed description of the preferred embodiments

The invention provides a biopotential signal acquisition system, comprising at least two active electrodes each for receiving a signal from a respective input. The input may for example be a dry electrode applied to the skin of a patient being monitored.

A unity gain buffer is used to feed forward a signal from an associated input. The buffered output is provided to the amplifiers of the active electrodes as a reference signal. The buffered signal is approximately equal to the input common mode signal so that the effective common mode input voltage appearing at the inputs of each amplifier is significantly reduced. This reduces the common mode gain of the active electrodes and thus improves their common mode rejection ratio while also avoiding saturation of the active electrodes.

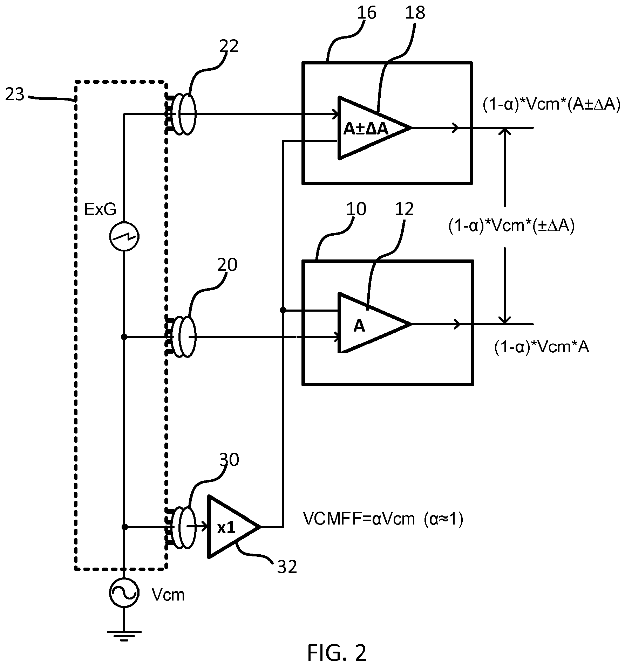

**Figure 2** shows a first example of signal acquisition system of the invention. The same reference numbers are used as in Figure 1 for the same components.

Instead of connecting one input of the amplifiers 12, 18 to a reference voltage as in Figure 1, a third electrode 30 and a unity gain voltage buffer 32 generate a feedforward voltage (VCMFF in Figure 2) approximately equal to the input CM signal (Vcm), which can be used to bias one input of the active electrode amplifiers 12,18.

As a result, the effective common-mode input voltage appearing at the inputs of each amplifier is significantly reduced. This method reduces the common-mode gain of the two active electrodes, and thus improves their CMRR and avoids the saturation of the active electrodes, while the differential amplification of the signal of interest ExG is not affected.

The signal levels are shown in Figure 2. These examples of signal levels are based on the common mode feedforward signal being applied to the inverting input of the amplifiers. For example, for the active electrode 16, the input signals are Vcm+ExG to the non-inverting input, and αVcm to the inverting input. The output is (A±ΔA)(Vcm+ExG- αVcm). The output signal resulting from the common mode interference is thus (A±ΔA)(1 - α)Vcm.

For the reference electrode 10, the signal levels shown are based on input signals Vcm to the non-inverting input, and αVcm to the inverting input. The output is (A)(Vcm - αVcm). The output signal all results from the common mode interference because this is the reference electrode, and the output resulting from the common mode interference is thus A(1 - α)Vcm. As will be clear from examples below, the common mode feedforward signal may instead be applied to the non-inverting input of the amplifiers, and the input signals applied to the inverting inputs. The final differential output signal remains the same.

This approach involves feeding forward a buffered common mode input signal to the other inputs of all amplifiers. Therefore, the inputs of each amplifier see a reduced common mode swing (of (1-α)*Vcm), instead of a full common mode swing of Vcm as in Figure 1. In this way, most of the input common mode interference is compensated before amplification.

The gain of the differential amplifier to the common mode input is reduced by a factor of 1/(1- α). As α approaches 1, this reduction in gain approaches infinity, corresponding to an infinite common mode rejection ratio. The large common mode signal is compensated at the inputs of each amplifier, and this means that amplifier saturation is avoided.

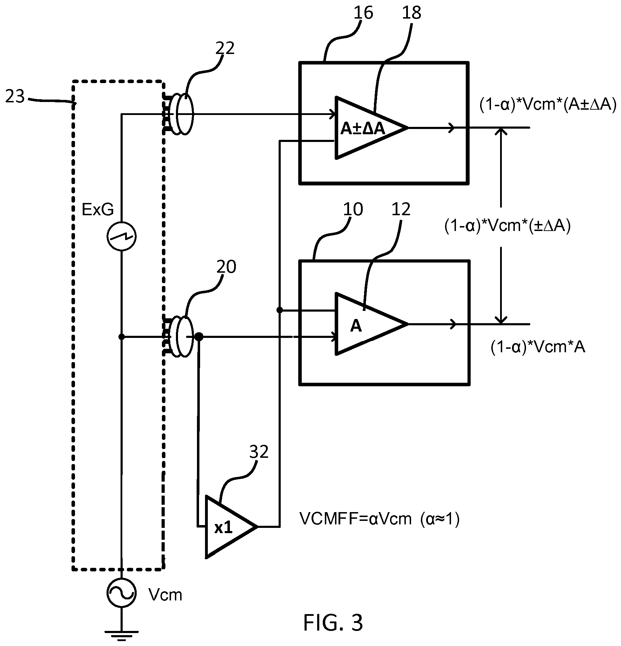

Figure 2 shows a system which has an input in the form of a skin contact 30 dedicated to the common mode buffer 32. However, the feedforward signal may be derived from one of the active electrode skin contacts, as shown in **Figure** 3. The contact 20 for the reference electrode is used to derive the common mode feedforward signal.

Figures 2 and 3 show single channel systems, with a reference active electrode and a single further active electrode. The approach can be extended to any number of active electrodes.

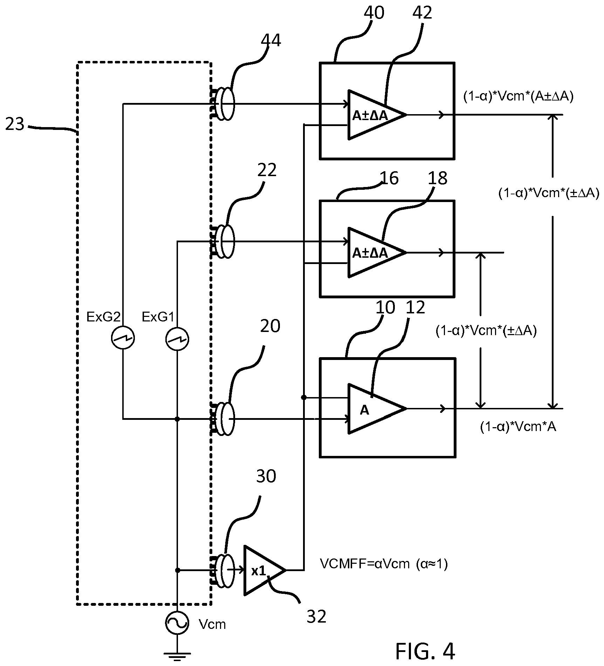

**Figure 4** shows the system extended with a third active electrode 40 with it associated amplifier 42. It receives a signal from a third input, again in the form of a patient contact 44. This is a two channel system, and the two biopotential signals being monitored are shown as ExG1 and ExG2. The common mode feedforward signal is applied to the inverting input of the amplifier of each of the three active electrodes. Both channels experience the same common mode rejection ratio if the value of ΔA is the same. Of course, for different values of ΔA for the two signal electrodes, the output signals resulting from the common mode interference will differ accordingly in dependence on the mismatch between the gain of the amplifier in the reference electrode 10 and in the signal electrodes 16, 42.

Figures 1 to 4 shows the signal levels at different points in the circuit which result from the common mode interfering signal Vcm. For simplicity, the signal of interest is not shown. In each case, it comprises an additional signal (A±ΔA)ExG at the output of the active signal electrode (or (A±ΔA)ExG1 and (A±ΔA)ExG2 for the two active signal electrodes in Figure 4).

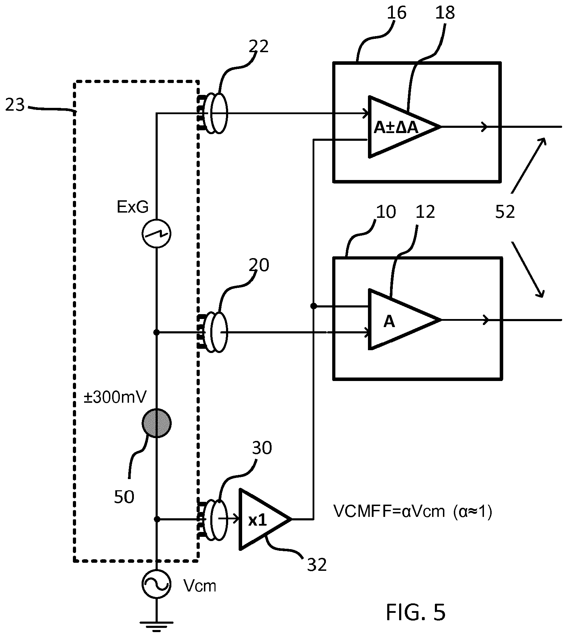

One possible drawback of the scheme in Figures 2 to 4 is that a DC signal which exists between the contacts is also fed forward to the active electrodes. This means, in practice that the electrode offset between leads can reach to ±300mV as shown in Figure 5. In **Figure 5****,** there is a DC offset between the contact 30 at which the common mode signal is derived and the contacts 20, 22. A large DC offset between the inputs of an active electrode amplifier can saturate the output, and cause the failure of ExG recording. In this case, the signals 52 become saturated.

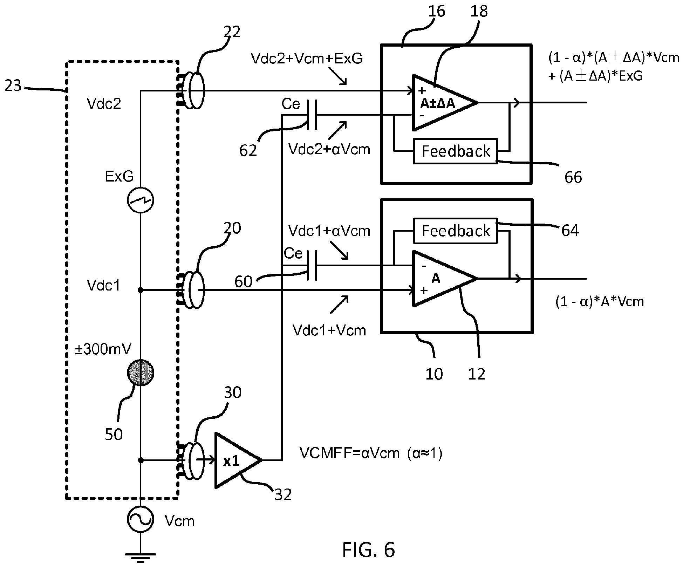

**Figure 6** shows an example modification to the circuit of Figure 2 in which the output from the buffer 32 is supplied to the inverting inputs of the amplifiers 12,18 through respective DC blocking capacitors 60,62.

Figure 6 shows different DC voltage levels Vdc1, Vdc2 at the patient contacts 20, 22 relative to the common mode voltage Vcm.

By capacitively coupling the fed forward common mode signal to each amplifier input, any DC voltage offset is cancelled so that only the AC components are coupled to the amplifier (such as 50 or 60Hz mains interference). The DC operating point after the capacitor 60, 62 is defined by a negative feedback loop 64, 66 together with capacitor 60, 62. This feedback loop with capacitor 60, 62 behaves as a low-pass filter, which only feeds back the DC and low frequency components to the inverting input of the amplifier. As a result, the DC voltage of the amplifier inverting input always follows its positive input. The electrode offset between leads is in this way blocked.

The DC voltage level at both inputs of the amplifier 12 is thus Vdc1 and the DC voltage level at both inputs of the amplifier 18 is Vdc2. The output signals resulting from the common mode interferer are the same as in Figure 2. The blocking capacitors 60,62 behave as a short circuit for the common mode signal. As discussed above, the effective common mode swing to each amplifier is reduced, which leads to improved CMRR of both active electrodes and avoid amplifier saturation.

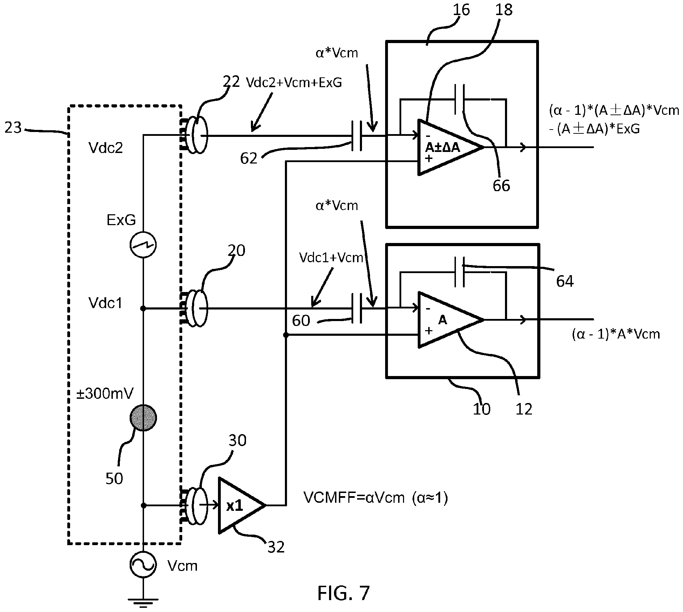

**Figure 7** shows an implementation in which the input signals are provided to the inverting inputs of the integrated amplifiers through the decoupling capacitors 60, 62, and the buffered common mode signal is provided to the non-inverting inputs. The same reference numbers are used as in Figure 6. The feedback paths are shown as implemented by capacitors 64, 66. Note that the common mode voltage across the input decoupling capacitors is almost zero so that there is no input common mode current. Thus, the common mode gain is reduced and the common mode rejection ratio is boosted.

In practice, the fed forward common mode signal will be connected to each active electrode through a cable which may be long. The buffered output is a low impedance node, which reduces the noise and interference pick-up from environment by the cable. Compared to a conventional common mode feedback scheme, the system has better stability and faster settling time because of the elimination of the feedback loop. There is also improved power/bandwidth tradeoff, as it is possible to use a low power analog buffer to achieve sufficient CMRR bandwidth (0.5-100Hz). In a conventional common mode feedback scheme, the feedback amplifier suffers from the tradeoff between bandwidth, power and stability. The power consumption of the feedback amplifier is usually high.

In a common mode feedback scheme, if one of the active electrodes is disconnected off, the whole feedback scheme does not work anymore. The approach described above allows each electrode to function regardless of the state of the others. The feedforward scheme can continuously run in the background without interrupting ExG signal recording.

The common mode feedforward approach has been verified by hardware measurement of a 4-channel digital active electrode system for EEG recording, and shows more than 30dB CMRR improvement and significant 50/60Hz interference reduction.

The invention is applicable generally to single or multi-channel ExG systems, especially for (digital) active electrode systems for healthcare monitoring. The invention does not require any particular active electrode design and can be applied regardless of the detailed architecture and implementation of the active electrodes.

Various other modifications will be readily apparent to those skilled in the art.

## Claims

1. A biopotential signal acquisition system for measuring biopotential signals (ExG) generated by a patient, comprising:
a first active electrode (10) comprising a first integrated amplifier (12); the first integrated amplifier (12) comprising a first input and a first buffered signal input, wherein the first input is connected to a first contact (20);
a second active electrode (16) comprising a second integrated amplifier (18); the second integrated amplifier (18) comprising a second input and a second buffered signal input, wherein the second input is connected to a second contact (22); and
a unity gain buffer (32) having an input connected to the first, the second or a third contact (20, 22, 30) and an output connected to the first buffered signal input of the first integrated amplifier (12) and to the second buffered signal input of the second integrated amplifier (18); and **is characterized in that** said biopotential signal acquisition system is configured such that, when the contacts are applied to the skin of the patient, said first and said second buffered signal input received by the first and the second integrated amplifier (12,18) contains a biopotential signal (ExG) generated by the patient.

2. A system as claimed in claim 1, wherein said first buffered signal input of the first integrated amplifier (12) and said second buffered signal input of the second integrated amplifier (18) are either both inverting or non-inverting inputs of said amplifiers.

3. A system as claimed in any preceding claim, further comprising at least a third active electrode (40) and comprising a third integrated amplifier (42).

4. A system as claimed in claim 3, wherein the unity gain buffer (32) provides the buffered output to the third integrated amplifier (42) as a reference common mode biopotential signal.

5. A system as claimed in any preceding claim, wherein the buffered common mode biopotential signal is provided to inverting inputs of the respective integrated amplifiers (12,18) through decoupling capacitors (60,62), and wherein the respective integrated amplifiers (12,18) comprise a negative feedback path (64,66).

6. A system as claimed in any one of claims 1 to 4, wherein the buffered common mode biopotential signal is provided to non-inverting inputs of the respective integrated amplifiers (12,18), wherein the first and second inputs are provided to the respective integrated amplifiers (12,18) through decoupling capacitors (60,62), and wherein the respective integrated amplifiers (12,18) comprise a negative feedback path (64,66).

7. A system as claimed in any preceding claim, wherein the first active electrode (10) comprises a reference electrode, and the second active electrode (16) comprises one of a set of active electrodes.

8. A system as claimed in claim 7, wherein the set comprises between 8 and 32 active electrodes.

9. An electronic circuit or device comprising a biopotential signal acquisition system as claimed in any of claims 1 to 8.

10. A biopotential signal acquisition method for measuring biopotential signals (ExG) generated by a patient by using a system according to claim 1, comprising:
capturing a first biopotential signal using a first active electrode (10) comprising a first integrated amplifier (12);
capturing a second biopotential signal using a second active electrode (16) comprising a second integrated amplifier (18);
buffering a common mode biopotential signal using a unity gain buffer (32) and **characterized by** providing, and the integrated amplifiers receiving, the buffered common mode biopotential signal, which also contains a biopotential signal (ExG) generated by the patient, to an input the first and second integrated amplifiers (12,18) as a reference common mode signal.

11. A method as claimed in claim 10, further comprising capturing a third biopotential signal using a third active electrode (40) comprising a third integrated amplifier (42), and providing the buffered common mode biopotential signal to the third integrated amplifier (42) as a reference common mode signal.

12. A method as claimed in claim 10 or 11, comprising providing the buffered common mode biopotential signal or the first and second biopotential signals to the respective integrated amplifiers (23,18) through decoupling capacitors (60,62), and providing the respective integrated amplifiers (12,18) with a negative feedback path.

## Patentansprüche

1. Biopotenzialsignalerfassungssystem zur Messung von Biopotentialsignalen (ExG), die von einem Patienten erzeugt werden, umfassend:
eine erste aktive Elektrode (10), die einen ersten integrierten Verstärker (12) umfasst; wobei der erste integrierte Verstärker (12) einen ersten Eingang und einen ersten gepufferten Signaleingang umfasst, wobei der erste Eingang mit einem ersten Kontakt (20) verbunden ist;
eine zweite aktive Elektrode (16), die einen zweiten integrierten Verstärker (18) umfasst; wobei der zweite integrierte Verstärker (18) einen zweiten Eingang und einen zweiten gepufferten Signaleingang umfasst, wobei der zweite Eingang mit einem zweiten Kontakt (22) verbunden ist; und
einen Puffer (32) mit Verstärkungsfaktor Eins, der einen Eingang, der mit dem ersten, dem zweiten oder einem dritten Kontakt (20, 22, 30) verbunden ist, und einen Ausgang aufweist, der mit dem ersten gepufferten Signaleingang des ersten integrierten Verstärkers (12) und mit dem zweiten gepufferten Signaleingang des zweiten integrierten Verstärkers (18) verbunden ist; und **dadurch gekennzeichnet, dass** das Biopotenzialsignalerfassungssystem so konfiguriert ist, dass, wenn die Kontakte an die Haut des Patienten angelegt werden, der erste und der zweite gepufferte Signaleingang, die von dem ersten und dem zweiten integrierten Verstärker (12, 18) empfangen werden, ein von dem Patienten erzeugtes Biopotentialsignal (ExG) enthalten.

2. System nach Anspruch 1, wobei der erste gepufferte Signaleingang des ersten integrierten Verstärkers (12) und der zweite gepufferte Signaleingang des zweiten integrierten Verstärkers (18) entweder beide invertierende oder nicht-invertierende Eingänge der Verstärker sind.

3. System nach einem vorstehenden Anspruch, das weiter mindestens eine dritte aktive Elektrode (40) umfasst und einen dritten integrierten Verstärker (42) umfasst.

4. System nach Anspruch 3, wobei der Puffer (32) mit Verstärkungsfaktor Eins den gepufferten Ausgang an den dritten integrierten Verstärker (42) als ein Referenz-Gleichtakt-Biopotentialsignal bereitstellt.

5. System nach einem vorstehenden Anspruch, wobei das gepufferte Gleichtakt-Biopotentialsignal den invertierenden Eingängen der jeweiligen integrierten Verstärker (12, 18) über Entkopplungskondensatoren (60, 62) bereitgestellt wird, und wobei die jeweiligen integrierten Verstärker (12, 18) einen negativen Rückkopplungspfad (64, 66) umfassen.

6. System nach einem der Ansprüche 1 bis 4, wobei das gepufferte Gleichtakt-Biopotentialsignal nicht-invertierenden Eingängen der jeweiligen integrierten Verstärker (12, 18) bereitgestellt wird, wobei die ersten und zweiten Eingänge den jeweiligen integrierten Verstärkern (12, 18) über Entkopplungskondensatoren (60, 62) bereitgestellt werden, und wobei die jeweiligen integrierten Verstärker (12, 18) einen negativen Rückkopplungspfad (64, 66) umfassen.

7. System nach einem vorstehenden Anspruch, wobei die erste aktive Elektrode (10) eine Referenzelektrode umfasst und die zweite aktive Elektrode (16) eine aus einem Satz von aktiven Elektroden umfasst.

8. System nach Anspruch 7, wobei der Satz zwischen 8 und 32 aktive Elektroden umfasst.

9. Elektronische Schaltung oder Vorrichtung umfassend ein Biopotenzialsignalerfassungssystem nach einem der Ansprüche 1 bis 8.

10. Biopotenzialsignalerfassungsverfahren zur Messung von Biopotentialsignalen (ExG), die von einem Patienten erzeugt werden, unter Verwendung eines Systems nach Anspruch 1, umfassend:
Erfassen eines ersten Biopotentialsignals unter Verwendung einer ersten aktiven Elektrode (10), die einen ersten integrierten Verstärker (12) umfasst;
Erfassen eines zweiten Biopotentialsignals unter Verwendung einer zweiten aktiven Elektrode (16), die einen zweiten integrierten Verstärker (18) umfasst;
Puffern eines Gleichtakt-Biopotentialsignals unter Verwendung eines Puffers (32) mit Verstärkungsfaktor Eins und **dadurch gekennzeichnet, dass** das gepufferte Gleichtakt-Biopotentialsignal, das auch ein vom Patienten erzeugtes Biopotentialsignal (ExG) enthält, an einen Eingang des ersten und des zweiten integrierten Verstärkers (12, 18) als ein Referenz-Gleichtaktsignal bereitgestellt wird und die integrierten Verstärker es empfangen.

11. Verfahren nach Anspruch 10, weiter umfassend das Erfassen eines dritten Biopotentialsignals unter Verwendung einer dritten aktiven Elektrode (40), die einen dritten integrierten Verstärker (42) umfasst, und das Bereitstellen des gepufferten Gleichtakt-Biopotentialsignals an den dritten integrierten Verstärker (42) als ein Referenz-Gleichtaktsignal.

12. Verfahren nach Anspruch 10 oder 11, umfassend das Bereitstellen des gepufferten Gleichtakt-Biopotentialsignals oder des ersten und zweiten Biopotentialsignals an die jeweiligen integrierten Verstärker (23, 18) über Entkopplungskondensatoren (60, 62) und das Bereitstellen der jeweiligen integrierten Verstärker (12, 18) mit einem negativen Rückkopplungspfad.

## Revendications

1. Système d'acquisition de signal biopotentiel pour mesurer des signaux biopotentiels (ExG) générés par un patient, comprenant :
une première électrode active (10) comprenant un premier amplificateur intégré (12) ; le premier amplificateur intégré (12) comprenant une première entrée et une première entrée de signal tamponné, dans lequel la première entrée est connectée à un premier contact (20) ;
une deuxième électrode active (16) comprenant un deuxième amplificateur intégré (18) ; le deuxième amplificateur intégré (18) comprenant une seconde entrée et une seconde entrée de signal tamponné, dans lequel la seconde entrée est connectée à un deuxième contact (22) ; et
un tampon à gain unitaire (32) présentant une entrée connectée au premier, au deuxième ou à un troisième contact (20, 22, 30) et une sortie connectée à la première entrée de signal tamponné du premier amplificateur intégré (12) et à la seconde entrée de signal tamponné du deuxième amplificateur intégré (18) ; et **est caractérisé en ce que** ledit système d'acquisition de signal biopotentiel est configuré de telle sorte que, lorsque les contacts sont appliqués sur la peau du patient, ladite première et ladite seconde entrée de signal tamponné reçue par le premier et le deuxième amplificateur intégré (12, 18) contiennent un signal biopotentiel (ExG) généré par le patient.

2. Système selon la revendication 1, dans lequel ladite première entrée de signal tamponné du premier amplificateur intégré (12) et ladite seconde entrée de signal tamponné du deuxième amplificateur intégré (18) sont toutes deux des entrées inverseuses ou non inverseuses desdits amplificateurs.

3. Système selon une quelconque revendication précédente, comprenant en outre au moins une troisième électrode active (40) et comprenant un troisième amplificateur intégré (42).

4. Système selon la revendication 3, dans lequel le tampon à gain unitaire (32) fournit la sortie tamponnée au troisième amplificateur intégré (42) en tant que signal biopotentiel en mode commun de référence.

5. Système selon une quelconque revendication précédente, dans lequel le signal biopotentiel en mode commun tamponné est fourni aux entrées inverseuses des amplificateurs intégrés respectifs (12, 18) par l'intermédiaire de condensateurs de découplage (60, 62), et dans lequel les amplificateurs intégrés respectifs (12, 18) comprennent un chemin de rétroaction négative (64, 66).

6. Système selon l'une quelconque des revendications 1 à 4, dans lequel le signal biopotentiel en mode commun tamponné est fourni aux entrées non inverseuses des amplificateurs intégrés respectifs (12, 18), dans lequel les première et seconde entrées sont fournies aux amplificateurs intégrés respectifs (12, 18) à travers des condensateurs de découplage (60, 62), et dans lequel les amplificateurs intégrés respectifs (12, 18) comprennent un chemin de rétroaction négative (64, 66).

7. Système selon une quelconque revendication précédente, dans lequel la première électrode active (10) comprend une électrode de référence, et la deuxième électrode active (16) comprend l'une parmi un ensemble d'électrodes actives.

8. Système selon la revendication 7, dans lequel l'ensemble comprend entre 8 et 32 électrodes actives.

9. Circuit ou dispositif électronique comprenant un système d'acquisition de signal biopotentiel selon l'une quelconque des revendications 1 à 8.

10. Procédé d'acquisition de signal biopotentiel pour mesurer des signaux biopotentiels (ExG) générés par un patient en utilisant un système selon la revendication 1, comprenant les étapes consistant à :
capturer un premier signal biopotentiel à l'aide d'une première électrode active (10) comprenant un premier amplificateur intégré (12) ;
capturer un deuxième signal biopotentiel à l'aide d'une deuxième électrode active (16) comprenant un deuxième amplificateur intégré (18) ;
tamponner un signal biopotentiel en mode commun à l'aide d'un tampon à gain unitaire (32) et **caractérisé par** la fourniture, et les amplificateurs intégrés recevant, le signal biopotentiel en mode commun tamponné, qui contient également un signal biopotentiel (ExG) généré par le patient, à une entrée des premier et deuxième amplificateurs intégrés (12, 18) comme signal en mode commun de référence.

11. Procédé selon la revendication 10, comprenant en outre la capture d'un troisième signal biopotentiel en utilisant une troisième électrode active (40) comprenant un troisième amplificateur intégré (42), et la fourniture du signal biopotentiel en mode commun tamponné au troisième amplificateur intégré (42) en tant que signal en mode commun de référence.

12. Procédé selon la revendication 10 ou 11, comprenant la fourniture du signal biopotentiel en mode commun tamponné ou des premier et deuxième signaux biopotentiels aux amplificateurs intégrés respectifs (23, 18) par l'intermédiaire de condensateurs de découplage (60, 62), et la fourniture aux amplificateurs intégrés respectifs (12, 18) avec un chemin de rétroaction négative.
